# EUROPEAN PATENT APPLICATION

(11) **EP 4 148 131 A1**
(43) Date of publication of application: **15.03.2023**
(21) Application number: 21196040.6
(22) Date of filing: 10.09.2021
(51) Int. Cl.: C12N 15/113, A61K 31/713

(54) **RNA FOR INHIBITING SARS-COV-2 REPLICATION**

(71) Applicant: Charité - Universitätsmedizin Berlin, 10117 Berlin (DE); Technische Universität Berlin, 10623 Berlin (DE); Freie Universität Berlin, 14195 Berlin (DE)
(72) Inventor: NIEMEYER, Daniela, 10245 Berlin (DE); KURRECK, Jens, 12205 Berlin (DE); TOLKSDORF, Beatrice, 13189 Berlin (DE); RÖHRS, Viola, 14469 Potsdam (DE); BERG, Johanna, 14197 Berlin (DE); KAUFER, Benedikt, 14532 Kleinmachnow (DE); LAUSTER, Daniel, 10179 Berlin (DE); TRIMPERT, Jakob, 10115 Berlin (DE); NIE, Chuanxiong, 13353 Berlin (DE)
(74) Representative: Maikowski & Ninnemann Patentanwälte Partnerschaft mbB

(57) **Abstract**

The invention relates to an RNA for inhibiting severe acute respiratory syndrome coronavirus 2 (SARS-CoV-2) replication. According to an aspect of the invention, the RNA is an at least partially double-stranded RNA having a sequence being at least 95 % identical to a sequence chosen from the group consisting of SEQ ID NO. 6 and SEQ ID NO. 11.

## Description

The present invention relates to an RNA for inhibiting severe acute respiratory syndrome coronavirus 2 (SARS-CoV-2) replication according to the preamble of claim 1, to (further) medical uses of such RNA according to the preambles of claim 2 and 4, to an in-vitro method for inhibiting SARS-CoV-2 replication in an isolated cell according to the preamble of claim 7, and to a pharmaceutical preparation according to the preamble of claim 8.

Coronavirus disease 2019 (COVID-19) is an infectious disease caused by the severe acute respiratory syndrome coronavirus 2 (SARS-CoV-2). The virus was first described in Wuhan, People's Republic of China, in December 2019 and spread worldwide very quickly. As of August 2021, approximately 200 million infections and more than four million fatalities have been reported worldwide.

Despite the remarkable development and approval of vaccines, satisfactory treatment options for patients with a severe course of disease are still lacking. So far only remdesivir, a ribonucleotide analogue inhibitor of viral RNA polymerase that was originally developed to treat Ebola infections but is also active against SARS-CoV-2 (Pruijssers et al., 2020), has received conditional approval by the European Medicines Agency (EMA) (Dal-Ré et al., 2021). Furthermore neutralizing monoclonal antibodies have been identified that block SARS-CoV-2 (Wan et al., 2020) and are therefore a class of biologics that is recommended for the treatment. Still, the development of effective antiviral drugs is urgently needed.

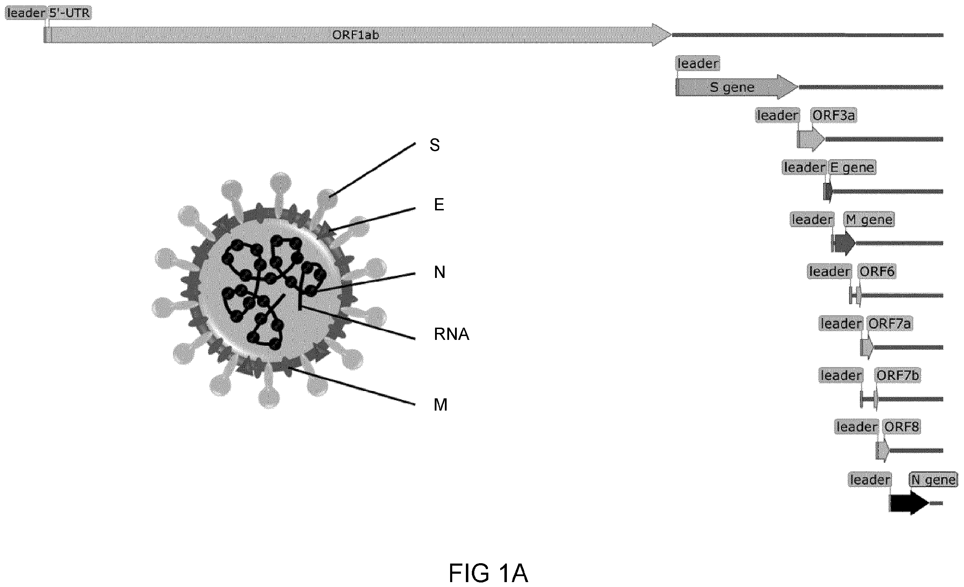

SARS-CoV-2 is an enveloped RNA virus with a high genomic homology to SARS-CoV-1 (82 %) and belongs to β-Coronavirus (Figure 1A). The single-stranded RNA genome (RNA) is of positive polarity, approximately 30 kilobases in length and codes for 16 non-structural proteins (nsp1-16) that are responsible for RNA replication, as well as for four structural proteins, namely the spike (S) protein, the envelope (E) protein, the membrane (M) protein and the nucleoprotein (N). The S, E and M proteins are embedded in the virus membrane that envelops the nucleocapsid, which is composed of the N protein and the virus genome (Chan et al., 2020; Pal et al., 2020).

The S protein is responsible for binding and entering the host cell and consists of two subunits (Walls et al., 2020). The S1 subunit contains the receptor binding domain (RBD), which conveys the binding to the host cell receptor angiotensin-converting enzyme 2 (ACE2). After cleavage by, e.g., the cellular protease transmembrane protease serine 2 (TMPRSS2), cell entry is subsequently mediated by the S2 subunit (Hoffmann et al., 2020).

Following cell entry, all proteins required for replication are directly translated (ORF1ab) and induce replication of the viral RNA genome (Figure 1A). During replication, negative sense RNA intermediates are produced to act as templates for positive-sense RNA synthesis (Romano et al., 2020). A unique feature of coronaviruses and other members of the order *Nidovirales* is that not only the complete genomic RNA but also subgenomic RNAs are produced (Figure 1A), which are essential for the translation of the structural proteins. These subgenomic RNAs all share the same 5'-end (and 3'-end) of around 75 nucleotides, the so-called leader sequence (Kim et al., 2020).

The current model of discontinuous transcription proposes that the viral RNA-dependent RNA polymerase (RdRp) switches template from the transcription regulatory sequences (TRS) that precede all of the ORFs, body-TRS, to the TRS in the 5'-untranslated region (5'-UTR), leader-TRS, during synthesis of the negative strand. This ultimately results in the fusion of the leader sequence to downstream parts of the genome. Subsequently, these negative-sense strand intermediates are transcribed into positive-sense strand subgenomic mRNAs (Sawicki and Sawicki, 1998). As soon as sufficient new RNA genomes and structural proteins are available, new viruses are formed and released (Schoeman and Fielding, 2019).

A promising approach to inhibit viral replication is the implementation of RNA interference (RNAi), which is a highly conserved, very efficient process of post-transcriptional gene silencing that is triggered by short double-stranded RNA, such as small interfering RNA (siRNA). RNAi leads to the degradation of mRNA in a sequence-specific manner and ultimately specifically inhibits the expression of genes (Bantounas et al., 2004; Kurreck, 2009; Hu et al., 2020).

Numerous RNAi-based therapies have been investigated for the treatment of viral infections in the past (Haasnoot and Berkhout, 2009; Levanova and Poranen, 2018). The inventors and others have demonstrated an effective reduction of target viral gene expression and virus replication, for example of the human immunodeficiency virus (Berkhout, 2009), influenza virus (Suzuki et al., 2009), coxsackievirus B3 (Werk et al., 2005; Stein et al., 2015), adenovirus (Schaar et al., 2017), hepatitis B virus (Chen et al., 2008) and hepatitis C virus (Chandra et al., 2012) in various cell and animal disease models. Following the emergence of SARS-CoV-1 in 2002, RNAi was one of the strategies pursued to inhibit SARS-CoV-1 replication. In several *in vitro* studies, it was shown that siRNAs efficiently reduced the expression of different target viral proteins and ultimately lead to the inhibition of viral replication (Wu et al., 2005; Chang et al., 2007).

Owing to its fundamental role in cell infection, the S protein of SARS-CoV-1 was considered the most promising target for inhibition with RNAi. However, the S protein is prone to mutations, which increase the virus fitness, as has also been observed for SARS-CoV-2 in a growing number of SARS-CoV-2 variants (e.g., alpha (B.1.1.7), beta (B.1.351), gamma (P.1) and delta (B.1.617.2) variants) (Jo et al., 2021). Obviously, mutations can alter the target sequence of siRNAs and severely reduce the efficiency of RNAi (Boden et al., 2003; Das et al., 2004; Wilson and Richardson, 2005). Therefore, targeting a strongly conserved region such as the 5'-UTR that is crucial for viral RNA replication and transcription was considered a promising target (Yang and Leibowitz, 2015; Baldassarre et al., 2020). By targeting the leader sequence of SARS-CoV-1, which is present in the viral genomic as well as subgenomic RNAs, Li *et al.* were able to effectively inhibit the expression of viral genes (S, E, M and N) and ultimately inhibit virus replication in Vero E6 cells (Li et al., 2005b). Moreover, the siRNA they developed targeting the leader sequence was more effective than the siRNA they developed in parallel targeting the S gene. Hence, the 5'-UTR of SARS-CoV-2 may be another promising target to develop RNAi-based therapies against COVID-19.

It is an object of the present invention to provide RNAs that are appropriate for inhibiting replication of SARS-CoV-2 and optionally of other coronaviruses.

This object is achieved with an RNA according to claim 1. Such an RNA is appropriate to inhibit severe acute respiratory syndrome coronavirus 2 (SARS-CoV-2) replication. The RNA is an at least partially double-stranded RNA and has a sequence being at least 95 %, in particular at least 97 %, in particular at least 98 %, in particular completely identical to a sequence chosen from the group consisting of SEQ ID NO. 6 and SEQ ID NO. 11. The SEQ ID NO. 6 describes a double-stranded small interfering RNA (siRNA) having a length of 21 nucleotides, wherein the two thymine nucleotides at its 3' terminus are single-stranded overlapping nucleotides. An identity of at least 95 % to SEQ ID NO. 6 means that at least 20 nucleotides of a specific RNA sequence are identical to SEQ ID NO. 6.

The SEQ ID NO. 11 describes a small hairpin RNA (shRNA) having a length of 57 nucleotides. It is double-stranded in dedicated sense/antisense regions and builds a hairpin loop in a loop region arranged between the sense and the antisense region. SEQ ID NO. 11 comprises a sense sequence corresponding to SEQ ID NO. 6 (but carrying thymine instead of uracil), an antisense sequence being complementary to SEQ ID NO. 6 (also carrying thymine instead of uracil), as well as a loop sequence between the sense sequence and the antisense sequence of SEQ ID NO. 6. Due to this structure, it is able to adopt the shape of a hairpin. Furthermore, since it incorporates both the sense and the antisense sequence of SEQ ID NO. 6, it is closely related to SEQ ID NO. 6. An identity of at least 95 % to SEQ ID NO. 11 means that at least 55 nucleotides of the sequence are identical to SEQ ID NO. 11.

In an embodiment, the sequence of the RNA is completely (to 100 %) identical to SEQ ID NO. 6 or SEQ ID NO. 11.

In the course of making the present invention, the inventors designed and tested eight siRNAs targeting the 5'-UTR of SARS-CoV-2. The most efficient siRNA, termed siCoV6 (corresponding to SEQ ID NO. 6), targets the leader sequence that is present in the viral genome as well as in all subgenomic fragments. When tested with infectious SARS-CoV-2, it effectively inhibited virus replication by two orders of magnitude (99 %) at a concentration of only 10 nM siRNA. As its target sequence is highly conserved, it was also active against the SARS-CoV-2 alpha variant and even against SARS-CoV-1 family member. This broad antiviral activity makes the siRNA a promising candidate for the treatment of SARS-coronavirus infections.

The sequences of the eight synthesized siRNAs, of a control RNA (siCon), as well as of three synthesized small hairpin RNAs (shRNAs) are listed in the following Table 1.

**Table 1. Sequences of designed siRNAs and shRNAs.**

| **siRNA** | **Sequence** | **SEQ ID NO.** |
|---|---|---|
| siCoV1 | 5'-UUAUACUGCGUGAGUGCACTT-3' 3'-TTAAUAUGACGCACUCACGUG-5' | 1 |
| siCoV2 | 5'-GAUAGACGAGUUACUCGUGTT-3' 3'-TTCUAUCUGCUCAAUGAGCAC-5' | 2 |
| siCoV3 | 5'-UUACCUUUCGGUCACACCCTT-3' 3'-TTAAUGGAAAGCCAGUGUGGG-5' | 3 |
| siCoV4 | 5'-UUACUCGUGUCCUGUCAACTT-3' 3'-TTAAUGAGCACAGGACAGUUG-5' | 4 |
| siCoV5 | 5'-AGUUACUCGUGUCCUGUCATT-3' | 5 |
| | 3'-TTUCAAUGAGCACAGGACAGU-5' | |
| siCoV6 | 5'-UUCGUUUAGAGAACAGAUCTT-3' 3'-TTAAGCAAAUCUCUUGUCUAG-5' | 6 |
| siCoV7 | 5'-UACCUUUCGGUCACACCCGTT-3' 3'-TTAUGGAAAGCCAGUGUGGGC-5' | 7 |
| siCoV8 | 5'-GAGAUCGAAAGUUGGUUGGTT-3' 3'-TTCUCUAGCUUUCAACCAACC-5' | 8 |
| siCon | 5'-ACGUGACACGUUCGGAGAATT-3' 3'-TTUGCACUGUGCAAGCCUCUU-5' | 9 |
| shCoV4 | 5'-GTTGACAGGACACGAGTAAgc CCTGACCCAgcTTACTCGTGTCCTGTCAAC TTTTTT-3' 3'-CCTAGGCAACTGTCCTGTGCTCATTcg GGACTGGGTcgAATGAGCACAGGACAGTTG AAAAAAGTTAACTTCGAA-5' | 10 |
| shCoV6 | 5'-GATCTGTTCTCTAAACGAAgc CCTGACCCAgcTTCGTTTAGAGAACAGATC TTTTTT-3' 3'-CCTAGGCTAGACAAGAGATTTGCTTcg GGACTGGGTcgAAGCAAATCTCTTGTCTAG AAAAAAGTTAACTTCGAA-5' | 11 |
| shCoV7 | 5'-GGGGTGTGACCGAAAGGTAgc CCTGACCCAgcTACCTTTCGGTCACACCCC TTTTTT-3' 3'-CCTAGGCCCCACACTGGCTTTCCATcg GGACTGGGTcgATGGAAAGCCAGTGTGGGG AAAAAAGTTAACTTCGAA-5' | 12 |

Each of the siRNAs and shRNAs listed above in Table 1 is herewith independently from other siRNAs or shRNAs claimed like siCoV6 (SEQ ID NO. 6) or shCoV6 (SEQ ID NO. 11). Thus, the present invention is not limited to RNAs corresponding to SEQ ID NO. 6 and SEQ ID NO. 11, but also extends to the other RNAs listed in Table 1 above. They can replace SEQ ID NOs. 6 and/or 11 in any application disclosed and/or claimed herein.

In an aspect, the present invention relates to the use of the RNA as a medicament.

In an embodiment, the medicament is an inhalation medicament, i.e., a medicament that is to be applied to a patient in need thereof by inhalation. For this purpose, the medicament can be nebulized so that a (fine) aerosol is prepared that can be easily inhaled by the patient and that efficiently transports the medicament to its site of action, in particular the patient's lung.

In an aspect, the present invention relates to the use of the RNA for treating a disease caused by a coronavirus. In this context, the coronavirus has a genome or subgenome comprising an RNA sequence being at least 95 %, in particular at least 97 %, in particular at least 98 % identical or complementary to a sequence chosen from the group consisting of SEQ ID NO. 6 and SEQ ID NO. 11.

In an embodiment, the coronavirus is a severe acute respiratory syndrome coronavirus (SARS-CoV). Presently, two types of SARS-CoV, namely SARS-CoV-1 and SARS-CoV-2 are known. Many variants or mutants of both SARS-CoVs are also known. Both SARS-CoV-1 and SARS-CoV-2 as well as the known variants or mutants comprise an RNA sequence in their leader sequence and their leader transcription regulatory sequence (TRS-L) that is fully complementary to SEQ ID NO. 6. Thus, this target sequence is - possibly due to its important function during replication of SARS-CoV highly conserved and well accessible. The inventors have so far not found any indications that the binding of an RNA corresponding to SEQ ID NO. 6 to the SARS-CoV target sequence is prevented by secondary or tertiary RNA structures. Therefore, SEQ ID NO. 6 is particularly appropriate for inhibiting SARS-CoV replication.

In an embodiment, the disease to be treated is coronavirus disease 2019 (COVID-19).

In an aspect, the present invention relates to an *in vitro* method for inhibiting SARS-CoV, in particular SARS-CoV-1 or SARS-CoV-2 replication in isolated cells. For this purpose, the method comprises the step of transfecting the isolated cells with an RNA according to the preceding explanations.

In an embodiment, the isolated cell is a eukaryotic cell, in particular a mammalian cell. Human cell lines like Hela cells or cell lines derived from the African green monkey like Vero cells, in particular Vero E6 cells, are particularly appropriate.

In an embodiment, the present invention relates to a pharmaceutical preparation comprising at least one RNA according to the preceding explanations.

In an embodiment, the pharmaceutical preparation additionally comprises an RNA having a sequence being at least 95 %, in particular at least 97 %, in particular at least 98 %, in particular completely identical to a sequence chosen from the group consisting of SEQ ID NO. 1, SEQ ID NO. 2, SEQ ID NO. 3, SEQ ID NO. 4, SEQ ID NO. 5, SEQ ID NO. 7, SEQ ID NO. 8, SEQ ID NO. 10, and SEQ ID NO. 12. Such a combination can be as efficient as the pure siCoV6 (SEQ ID NO. 6), but can reduce the risk or even prevent the formation of novel SARS-CoV mutants.

In an embodiment, the pharmaceutical preparation comprises an RNA having a sequence being at least 95 %, in particular at least 97 %, in particular at least 98 %, in particular completely identical to SEQ ID NO. 11 and an RNA having a sequence being at least 95 %, in particular at least 97 %, in particular at least 98 %, in particular completely identical to a sequence chosen from the group consisting of SEQ ID NO. 10 and SEQ ID NO. 12. Thus, in this embodiment, the pharmaceutical preparation comprises not only a single shRNA, but rather two or three shRNAs, namely, shCoV6 combined with shCoV4 and/or shCoV7. Such a combination of different small hairpin RNAs prevents a viral escape due to mutations of the coronavirus since it efficiently targets different RNA sequences of the coronavirus genome. A combination of SEQ ID NO. 11 with SEQ ID NO. 12 turned out to be particular efficient in inhibiting replication of SARS-CoV-2.

In an embodiment, the pharmaceutical preparation further comprises an adeno-associated virus (AAV) vector. AAV vectors are well-established and reliable vehicles for transporting RNA into cells, such as cells of a living organism like a human patient. Thus, AAV vectors can be used as transporter for any of the previous mentioned RNAs, in particular of the shRNAs.

In an embodiment, the pharmaceutical preparation is to be applied to a patient by means of inhalation. Thus, an appropriate dosage form of the pharmaceutical preparation is an inhalation-suited dosage form like an aerosol.

In an aspect, the present invention relates to a medical method for treating or preventing a disease caused by a coronavirus in a person in need thereof. In this context, the coronavirus has a genome or subgenome comprising an RNA sequence being at least 95 % identical or complementary to a sequence chosen from the group consisting of SEQ ID NO. 6 and SEQ ID NO. 11. The method comprises administering to the person a pharmaceutically active amount of an at least partially double-stranded RNA having a sequence being at least 95 % identical to a sequence chosen from the group consisting of SEQ ID NO. 6 and SEQ ID NO. 11.

All embodiments of the RNA can be combined in any desired way and can be transferred either individually or in any arbitrary combination to the uses, to the methods, and to the pharmaceutical preparation. Likewise, all embodiments of the uses can be combined in any desired way and can be transferred either individually or in any arbitrary combination to the RNA, to the methods, and to the pharmaceutical preparation. Furthermore, all embodiments of the methods can be combined in any desired way and can be transferred either individually or in any arbitrary combination to the RNA, to the uses, and to the pharmaceutical preparation. Finally, all embodiments of the pharmaceutical preparation can be combined in any desired way and can be transferred either individually or in any arbitrary combination to the RNAs, to the uses, and to the methods. In particular, the described uses, methods as well as the described pharmaceutical preparation are also applicable for the RNAs listed in Table 1 beyond the RNAs having a sequence corresponding to SEQ ID NO. 6 and SEQ ID NO. 11.

Further details of aspects of the present invention will be explained in the following making reference to exemplary embodiments and accompanying Figures. In the Figures:
- Figure 1A: shows a schematic overview of the SARS-CoV-2 genome;
- Figure 1B: shows a schematic overview of the target sequences of the siRNAs (SEQ ID NO. 1 to SEQ ID NO. 8) in the 5'-UTR;
- Figure 2A: shows a schematic representation of the reporter plasmid psiCheck2;
- Figure 2B: shows efficient targeting of the 5'-UTR of SARS-CoV-2 by the designed siRNAs;
- Figure 3A: shows the inhibition of SARS-CoV-2 replication by siCoV6 at different time points after infection;
- Figure 3B: illustrates the observed cell morphology in the experiments of Figure 3A;
- Figure 4A: shows the inhibition of SARS-CoV-2 alpha and SARS-CoV-1 replication by siCoV6;
- Figure 4B: illustrates the observed cell morphology of SARS-CoV-2 alpha in the experiments of Figure 4A;
- Figure 4C: illustrates the observed cell morphology of SARS-CoV-1 in the experiments of Figure 4A;
- Figure 5A: shows the sequence of shCoV4 (SEQ ID NO. 10) with 3' and 5' restriction enzyme cleavage sites;
- Figure 5B: shows the sequence of shCoV6 (SEQ ID NO. 11) with 3' and 5' restriction enzyme cleavage sites;

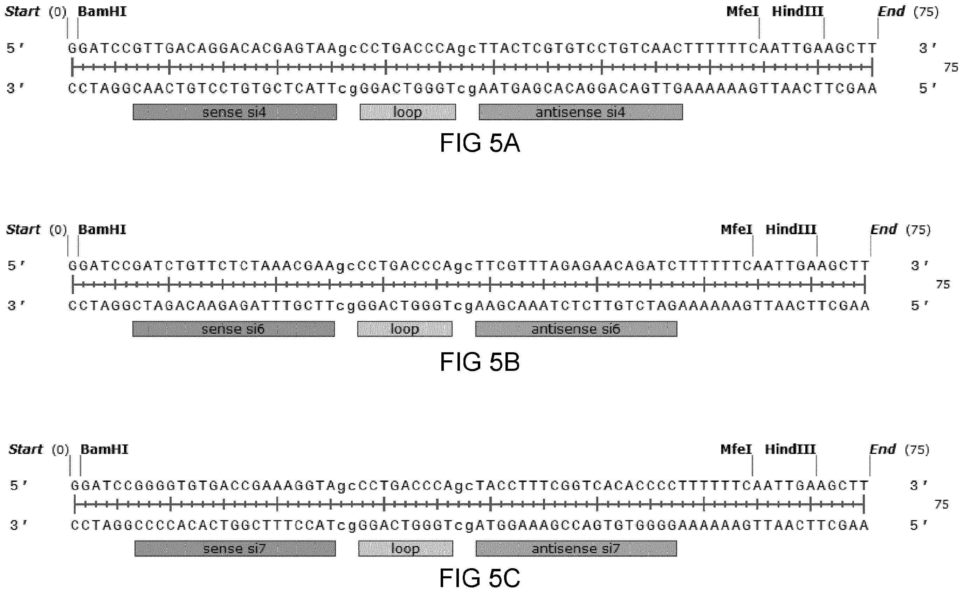

- Figure 5A: shows the sequence of shCoV7 (SEQ ID NO. 12) with 3' and 5' restriction enzyme cleavage sites;

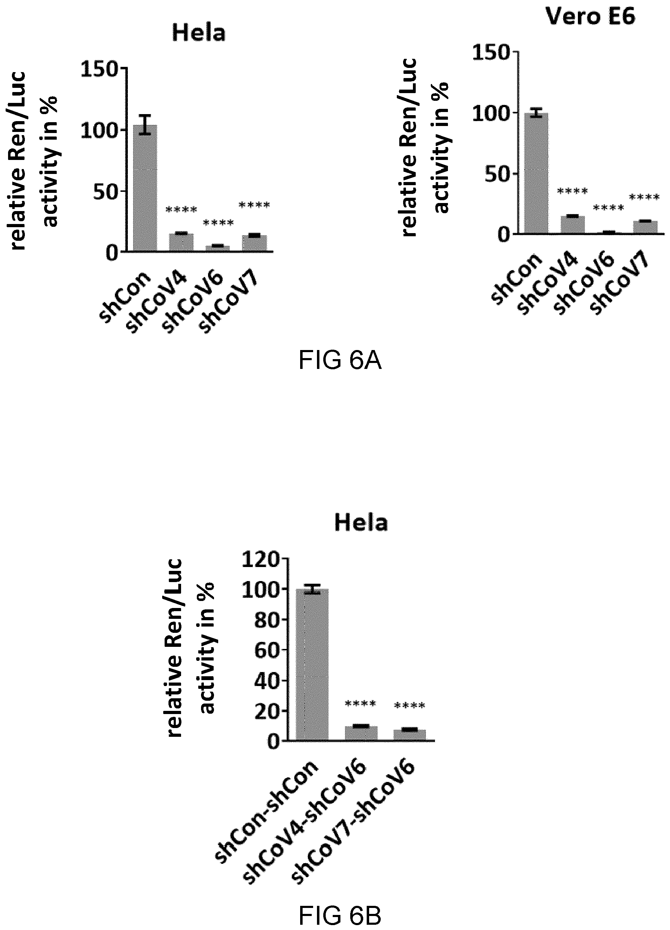

- Figure 6A: shows an efficiency comparison of the different shRNAs against the 5'-UTR of SARS-CoV-2 by a luciferase assay; and
- Figure 6B: shows an efficiency comparison of different shRNAs combinations against the 5'-UTR of SARS-CoV-2 by a luciferase assay.

Figure 1A was already briefly discussed in the introduction. It shows a schematic overview of the SARS-CoV-2 genome, the subgenomic RNAs and the virion structure. The coronavirus virion consists of the structural spike protein (S), envelope protein (E), membrane protein (M) and nucleoprotein (N). The single-stranded RNA genome (RNA) with positive polarity is encapsulated by N, while S-trimers protrude from the host-derived virus envelope and enable binding to new host cells. In addition to the genomic RNA, nine subgenomic RNAs are synthesized during replication, all of which share the same 5'- and 3'-ends. The term "leader" represents the mutual 5'-end, the leader sequence.

The other Figures will be explained in the following in the context of exemplary embodiments.

### Design of siRNAs targeting the 5'-UTR of SARS-CoV-2

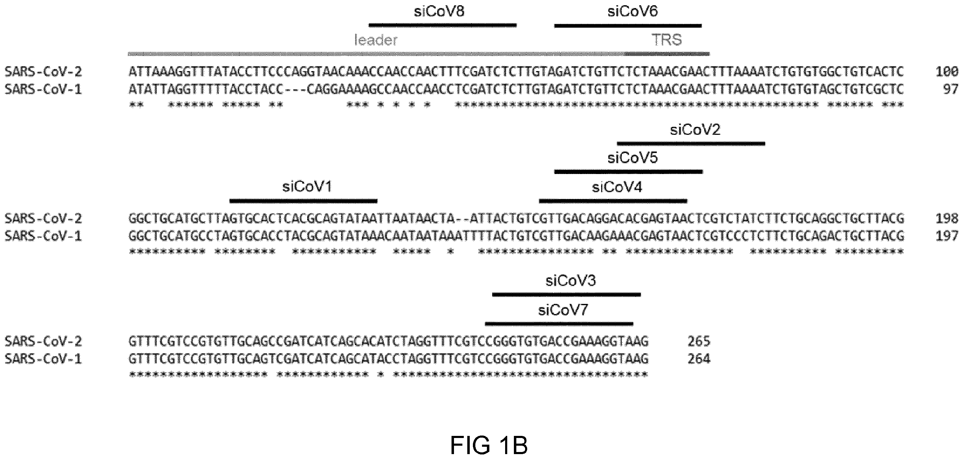

The 5'-UTR of SARS-CoV-2 is a conserved region that is crucial for viral RNA replication and transcription, which makes it a promising target for the development of RNAi-based therapies against COVID-19 (Yang and Leibowitz, 2015; Baldassarre et al., 2020). As the efficiency of siRNAs strongly depends on their thermodynamic design and the structure of the target region (Kurreck, 2006), eight siRNAs targeting the 5'-UTR of SARS-CoV-2 (NCBI RefSeq NC_045512.2) were designed *in silico* (Figure 1B, Table 1) using the online tool Horizon siDESIGN Center. Three of these siRNAs (siCoV3, siCoV6, siCoV7) bind to highly conserved regions that are completely homologous to SARS-CoV-1, cf. Figure 1B illustrating the target sequences of the siRNAs in the 5'-UTR of SARS-CoV-2. The leader sequence (leader) and the transcription regulatory sequence (TRS) can be found at the 5'-end of the genomic and subgenomic RNAs. Nucleotides without an asterisk differ from the SARS-CoV-1 5'-UTR sequence. The absence of seed sequences in the human transcriptome was ensured by the Nucleotide BLAST program using default parameters. The control siRNA siCon does not match any sequence present in the viral or human genome (Eckstein et al., 2010).

### RNAi-mediated targeting of the 5'-UTR of SARS-CoV-2 in reporter assays

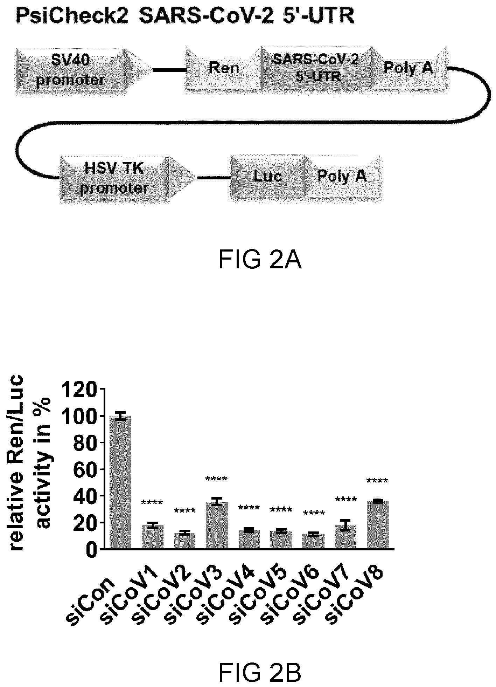

The silencing activity of the designed siRNAs was determined in a dual luciferase reporter assay. For the assay, the reporter plasmid psiCheck2 SARS-CoV-2 5'-UTR (Figure 2A) expressing the *Renilla-*luciferase reporter gene fused with the 5'-UTR of SARS-CoV-2 was used. Hela cells were transiently co-transfected with 250 ng of the reporter plasmid and 40 nM of one of the eight different siRNA candidates to evaluate their silencing activity. The relative Ren/Luc-activity was determined 48 h after transfection. For this purpose, the relative Ren/Luc-activity was determined and the negative control (siCon) was set to 100 %. The mean ± SEM (standard error of the mean) of three independent experiments is shown. The statistical significance was determined by a univariate analysis of variance (one-way ANOVA), wherein **** denotes p < 0.0001.

The expression of *Renilla-*luciferase was significantly inhibited by all eight siRNAs, with siCoV6, siCoV2, siCoV5 and siCoV4 achieving inhibition rates of around 90 % (Figure 2B). These results demonstrate that the 5'-UTR of SARS-CoV-2 can be efficiently targeted by a number of different siRNAs. The inventors chose the most effective candidate, siCoV6, which binds to the TRS of the leader sequence, for the subsequent tests.

### RNAi-mediated inhibition of SARS-CoV-2 replication

After having shown that the siRNA is active against the SARS-CoV-2 5'-UTR in reporter assays, the inventors tested siCoV6 against the infectious SARS-CoV-2 virions in Vero E6 cells. This siRNA is particularly promising, as it targets the TRS in the leader sequence and is thus directed against the genomic and subgenomic RNA transcripts. It can therefore be expected to be particularly efficient, as the RNAi approach should degrade not only the genomic RNA but also all subgenomic mRNAs.

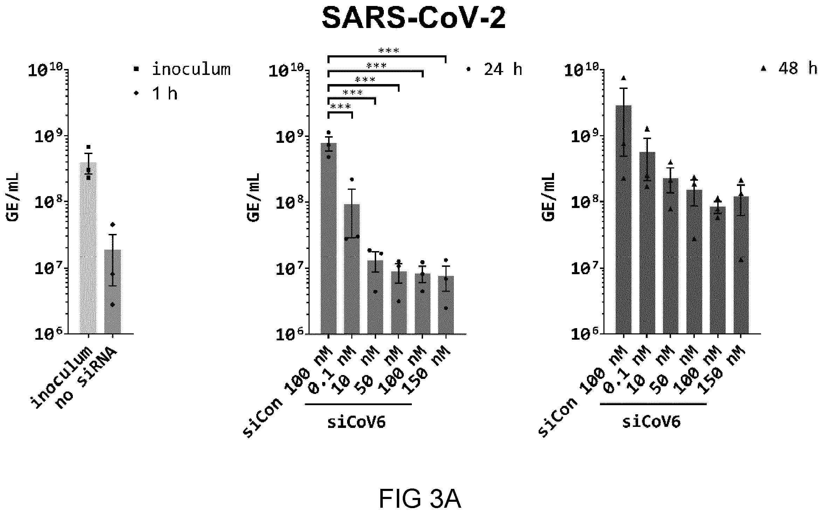

For the virus inhibition assays, Vero E6 cells were transfected with varying concentrations of siCoV6 (0.1 nM to 150 nM) and infected with SARS-CoV-2 at a multiplicity of infection (MOI) of 0.01 after 24 h. Subsequently, the cells were washed, supplemented media was added, and viral RNA was isolated from the culture supernatant at 1 hour post infection (hpi), 24 hpi and 48 hpi. Genome equivalents per mL (GE/mL) were determined by quantitative RT-PCR (Corman et al., 2020). The mean ± SEM of three independent experiments is shown in Figure 3A. The statistical significance was determined by a univariate analysis of variance (one-way ANOVA), wherein *** denotes p < 0.001.

The tested concentrations of siCoV6 led to a significant inhibition of the replication of SARS-CoV-2 after 24 h in a concentration-dependent manner (Figure 3A). In the control infection (100 nM siCon), 7.91×10⁸ GE/mL were detected, while already the lowest tested concentration of 0.1 nM siCoV6 resulted in a reduction of approximately one order of magnitude to 9.37×10⁷ GE/mL. The most effective inhibition was seen at the highest concentration of 150 nM siCoV6, with a reduction to less than 1 % of the control value to 7.64×10⁶ GE/mL.

The extent of virus inhibition decreased at 48 h post infection. The detected genomic equivalents were still reduced by up to 97 %, for example to 8.36×10⁷ GE/mL with 100 nM siCoV6 compared to 2.85×10⁹ GE/mL that were measured for the control. However, the reduction no longer reached statistical significance.

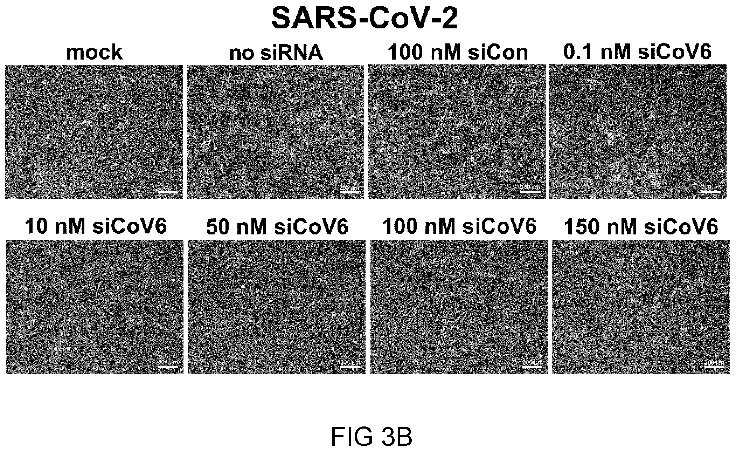

The protective effect of the siRNA treatment was further investigated by documenting the cytopathic effect (CPE). For this purpose, cells were fixed 48 h after infection with SARS-CoV-2 and the CPE of viral infection was documented by transmitted light microscopy. Representative data from three independent experiments are shown in Figure 3B. The scale bar indicates 200 µm. As expected, no CPE was visible in mock-infected control cells (Figure 3B). In clear contrast, infection with SARS-CoV-2 in untreated cultures (no siRNA) and cultures treated with the control siRNAs (siCon) induced visible CPE characterized by rounding of the cells and detachment from the cell culture plate. In line with the observed RNAi-mediated inhibition of SARS-CoV-2 with siCoV6, siRNA-treatment strongly reduced the CPE. In particular, a concentration of 50 nM siCoV6 or more almost completely inhibited the onset of CPE. These results clearly demonstrate that siCoV6 inhibits replication of SARS-CoV-2 in a concentration-dependent manner and protects cells from infection-induced cell toxicity.

### Inhibition of the SARS-CoV-2 alpha variant by siCoV6

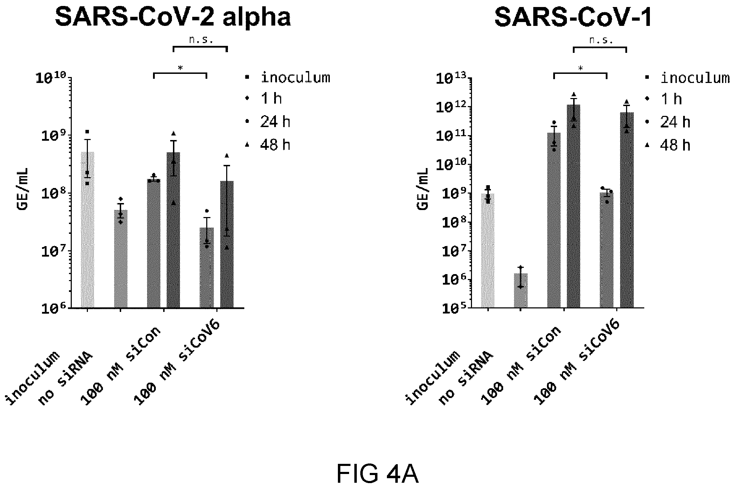

Like other viruses, SARS-CoV-2 is constantly evolving, as seen by a growing number of SARS-CoV-2 variants (Jo et al., 2021). Mutations can result in the alteration of siRNA target sequences and severely reduce the efficiency of RNAi (Boden et al., 2003; Das et al., 2004; Wilson and Richardson, 2005). Regions of the viral genome that are highly conserved are less likely to accumulate mutations that are not detrimental to virus replication and are preferred target sites for siRNAs. Therefore, three of the siRNAs (siCoV3, siCoV6, siCoV7) were designed to target highly conserved regions of the coronavirus genome (Figure 1B). One of the siRNAs, siCoV6, was specifically designed against the highly conserved TRS in the leader sequence. It can therefore be expected to be not only very efficient as it targets the genomic RNA as well as all subgenomic transcripts, but also to be active against SARS-CoV-2 variants. To test this hypothesis, the inventors next examined whether siCoV6 can also inhibit the replication of the SARS-CoV-2 alpha variant. To this end, Vero E6 cells were transfected with 100 nM of siCoV6 (or 100 nM siCon) and infected 24 h after transfection with SARS-CoV-2 alpha or SARS-CoV-1 at a MOI of 0.01. Genome equivalents per mL (GE/mL) were determined as described above (Niemeyer et al., 2018; Corman et al., 2020). The mean ± SEM of three independent experiments is shown in Figure 4A. The statistical significance was determined by Student's *t*-test, wherein * denotes p < 0.05.

As can be seen in Figure 4A, siRNA-treatment of the cells inhibited replication of the SARS-CoV-2 alpha variant significantly by approximately 7-fold from 1.78×10⁸ GE/mL in the control culture to 2.54×10⁷ GE/mL in the treated culture 24 h post infection. As already observed for SARS-CoV-2, the inhibitory activity became less prominent at 48 h post infection. The virus load was reduced by 70 % from 5.02×10⁸ GE/mL in the cells treated with the control siRNA to 1.61×10⁸ GE/mL in cells treated with the active siCoV6.

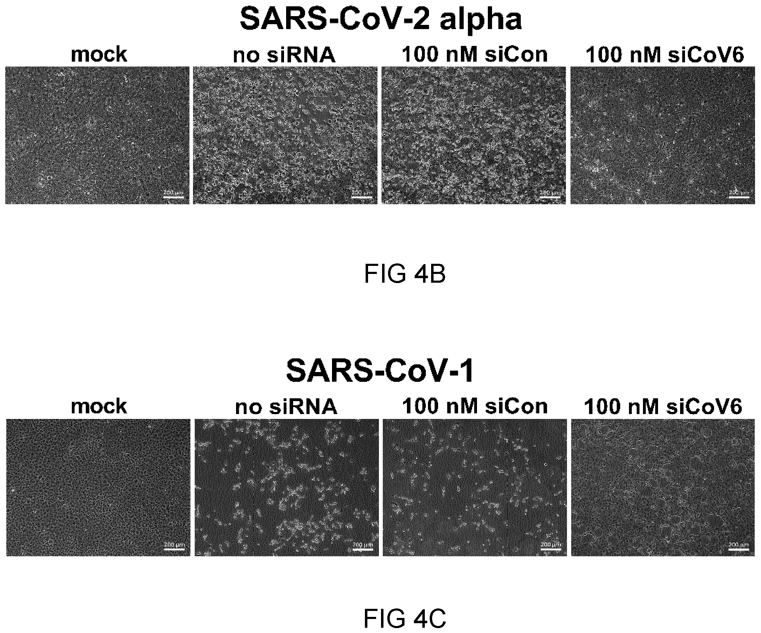

The inventors also documented the CPE 48 h post infection by light microscopy. Representative data from three independent experiments are shown Figure 4B, wherein the scale bar indicates 200 µm. As expected, no CPE became visible in the mock-infected control cells, while infection with the SARS-CoV-2 alpha variant induced a strong CPE in untreated (no siRNA) and siCon-treated cells. In sharp contrast, as already observed for SARS-CoV-2, siCoV6 strongly reduced the occurrence of a CPE by the infection of SARS-CoV-2 alpha variant.

Currently, other SARS-CoV-2 variants, in particular the delta variant, become predominant in many countries worldwide. The inventors therefore carried out a bioinformatic analysis of approximately 1.500 sequences of different SARS-CoV-2 variants (NCBI Virus SARS-CoV-2 Data Hub, as of June 2021) with respect to mutations in the target site of siCoV6. In this analysis, the inventors did not identify any mutations in either the SARS-CoV-2 beta or delta variant. For the alpha and gamma variants, only less than 0.8 % of the isolates had a mutation in the target site of siCoV6, but the important seed region of siCoV6 was never affected. This confirms that the targeted TRS is particularly well-suited for the development of a broad-range RNAi therapeutic.

### Inhibition of SARS-CoV-1 by siCoV6

As siCoV6 targets a sequence which is conserved between SARS-CoV-1 and SARS-CoV-2 (Figure 1B), the inventors reasoned that it may also be active against the first SARS-coronavirus. The inventors therefore tested the inhibitory efficiency of siCoV6 against SARS-CoV-1 under the same conditions as in their previous experiments. As can be seen in Figure 4A, RNAi treatment inhibited virus replication by approximately two orders of magnitude to 1.06×10⁹ GE/mL compared to the control in which 1.24×10¹¹ GE/mL were determined after 24 h. Again, the effect substantially decreased after 48 h and no longer reached significant levels.

The inventors furthermore investigated the CPE 48 h post infection by light microscopy (Figure 4C). As expected, mock infected control cells showed no sign of CPE, while infection with SARS-CoV-1 resulted in the cytopathic phenotype observed before in untreated (no siRNA) and siCon-treated cultures. In contrast, as already observed for SARS-CoV-2 and the SARS-CoV-2 alpha variant, siCoV6 strongly reduced the occurrence of a CPE.

Taken together, the inventors demonstrated that an siRNA designed to target the TRS in the 5'-UTR of SARS-coronaviruses is highly active against SARS-CoV-2, including its variant alpha. Importantly, a substantial inhibitory effect was also observed for the more distantly related SARS-CoV-1; the presently described siRNA might therefore have the potential to be developed into an anti-coronavirus therapy with a broad target range.

### Inhibition of SARS-CoV-2 replication by shRNA

To enable RNA delivery by adeno-associated viruses (AAV) vectors, shRNAs were derived from the most efficient siRNAs siCoV4, siCoV6 and siCoV7. An shRNA is a double-stranded RNA molecule that, by linking the antisense and sense sequences of an siRNA through a loop, forms a hairpin structure. To prevent viral escape by mutations, two or more different shRNAs can be used simultaneously.

The sequence of the synthesized shRNAs with 3' and 5' restriction enzyme cleavage sites is illustrated in Figures 5A (shCoV4), 5B (shCoV6), and 5C (shCoV7). The sense, antisense and intermediate loops regions are indicated. The sequences illustrated in Figures 5A to 5C comprise restriction enzyme recognition sites at their 3' end and their 5' end (indicated by the name of the respective restriction enzymes, i.e., BamHI, MfeI and HindIII). In the final shRNAs, these recognition sites will not be present anymore.

The efficiency of the shRNAs was also investigated using a luciferase assay as described above. For this purpose, plasmids containing the shRNAs individually or in combination were co-transfected with the reporter plasmid psiCheck-2 SARS-CoV-2 5'-UTR in Hela or Vero E6 cells, respectively. For this purpose, Hela and Vero E6 cells were co-transfected with 250 ng of the reporter plasmid and 250 ng of the respective shRNA plasmid (Figure 6A) or 250 ng of the respective 2xshRNA plasmid (Figure 6B). The proteins were extracted 48 h after transfection and relative Ren/Luc activity was determined. The respective negative controls were set to 100 %. The mean ± SEM is shown. The statistical significance was determined by univariate analysis of variance (one-way anova), wherein **** denotes p < 0,0001. All shRNAs examined were able to significantly downregulate Ren/Luc activity, again with shCoV6 being the most efficient with a 95 % downregulation in Hela or 98 % in Vero E6 (Figure 6A).

### Materials and methods

The following Table 2 lists the materials used in the experiments explained above. The applied methods will be briefly explained in more detail in the sections following after Table 2.

**Table 2: Used materials**

| **REAGENT or RESOURCE** | **SOURCE** | **IDENTIFIER** |
|---|---|---|
| **Chemicals** | | |
| Formaldehyde | Roth | Cat# 7398.1 |

| **Probes and primer** | | |
|---|---|---|
| SARS-CoV-2 | | |
| E-Sarbeco_P1 | TIB Molbiol | FAM-ACACTAGCCATCCTTAC TGCGCTTCG-BBQ |
| E_Sarbeco_F | Thermo Fisher Scientific | 5'-ACAGGTACGTTAATAGTTA ATAGCGT-3' |
| E_Sarbeco_R | Thermo Fisher Scientific | 5'-ATATTGCAGCAGTACGCAC ACA-3' |
| SARS-CoV-1 | | |
| SARS-P | TIB Molbiol | Fam-5'-ACGTTCGTGCGTGGA TTGGCTTTG-3'-BHQ |
| SARS-F | Thermo Fisher Scientific | 5'-CCCGCGAAGAAGCTATTC G-3' |

| SARS-R | Thermo Fisher Scientific | 5'-AGTTGCATGACAGCCCTCT ACA-3' |
|---|---|---|
| **siRNAs** | | |
| siRNAs | Eurofins Genomics | sequences see Table 1 |

| **Plasmids** | | |
|---|---|---|
| psiCHECK^{™}-2 | Promega | Cat# C8021 |

| **Enzymes** | | |
|---|---|---|
| Notl | NEB | Cat# R0189S |
| Xhol | NEB | Cat# R0146S |

| **Media** | | |
|---|---|---|
| DMEM | Biowest | Cat# L0101 |
| optiPRO | Thermo Fisher Scientific | Cat# 12309019 |
| FCS | c.c.pro | Cat# S-14-L |
| Sodium pyruvate | Sigma | Cat# S8636 |
| NEAA | Biowest | Cat# X0557 |
| Penicillin/streptomycin | Biowest | Cat# L0022 |
| L-Glutamin | Biowest | Cat# X0550 |

| **Transfection reagents** | | |
|---|---|---|
| Lipofectamin 2000 | Thermo Fisher Scientific | Cat# 11668027 |
| Lipofectamin RNAiMAX | Thermo Fisher Scientific | Cat# 13778075 |

| **Critical commercial assays** | | |
|---|---|---|
| Dual-Luciferase Reporter Assay System | Promega | Cat# E1980 |
| innuPREP Virus TS RNA Kit | Analytik Jena | Cat# 845-KS-4710250 |
| NucleoSpin RNA Virus | Macherey-Nagel | Cat# 740956.50 |
| SuperScript III One-Step RT-PCR System with Platinum Taq DNA Polymerase | Thermo Fisher Scientific | Cat# 12574018 |

| **Viruses** | | |
|---|---|---|
| SARS-CoV-2 | Laboratory of Jakob Trimpert | BetaCoV/Munich/BavPat2-ChVir984-ChVir1017/2020 |
| SARS-CoV-2 alpha variant | Laboratory of Jakob Trimpert | BetaCoV/Germany/ChVir21652/20 20 |
| SARS-CoV-1 | Laboratory of Christian Drosten | recombinant SARS-CoV-1, GenBank: AY310120.1 (Pfefferle et al., 2009) |

| **Cell lines** | | |
|---|---|---|
| Vero E6 | ATCC | CRL-1586 |
| Hela | DSMZ | ACC 57 |

| **Software, algorithms and databases** | | |
|---|---|---|
| Horizon siDESIGN Center | Horizon | https://horizondiscovery.com/en/or dering-and-calculation-tools/sidesign-center |
| GraphPad Prism 8 | GraphPad | |
| Nucleotide BLAST | NIH | https://blast.ncbi.nlm.nih.gov/Blast. cgi?PAGE_TYPE=BlastSearch |
| SARS-CoV-2 Data Hub | NCBI Virus | https://www.ncbi.nlm.nih.gov/labs/v irus/vssi/#/virus?SeqType_s=Nucle otide&VirusLineage_ss=SARS-CoV-2, %20taxid:2697049 |

### Design of siRNA and plasmid construction

Eight siRNA duplexes targeting the 5'-UTR of SARS-CoV-2 (Figure 1B) were designed *in silico* using Horizon siDESIGN Center. The absence of seed sequences in the human transcriptome was ensured by the Nucleotide BLAST program using default parameters. The control siRNA siCon does not match any sequence present in the viral or human genome (Eckstein et al., 2010). All siRNAs were purchased from Eurofins Genomics (Ebersberg, Germany) and their sequences are shown in Table 1.

The shRNA vectors were generated by connecting the DNA sequence encoding the sense siRNA strand to the DNA sequence encoding the antisense siRNA strand using the loop 5'-CCTGACCCA-3' (Figure 2E). All DNA sequences were synthesized by Thermo Fisher Scientific and cloned into the BamHI/HindIII-digested pscAAV-GFP (Fechner et al., 2008). The sequences can be found in Table 1. Double shRNA-expressing vectors were cloned following a building block principle based on the restriction sites EcoRI and MfeI as previously described (Rothe et al., 2010). Shortly, the donor plasmid was digested with MfeI/EcoRI and the resulting fragment subsequently inserted into the acceptor plasmid, which was linearized by EcoRI and already contained a shRNA expression cassette.

The silencing efficacy of the siRNAs was determined in reporter assays using a vector expressing the firefly luciferase reporter gene fused with the 5'-UTR of SARS-CoV-2. The 5'-UTR of SARS-CoV-2 (NCBI RefSeq NC_045512.2) was synthesized by Thermo Fisher Scientific and inserted downstream the *Renilla-*luciferase gene into the psiCHECK^{™}-2 Vector (Promega, Walldorf, Germany) plasmid using the restriction sites XhoI/NotI, giving rise to psiCheck2 SARS-CoV-2 5'-UTR.

### Cell culture

Vero E6 cells (CRL-1586, ATCC, Manassas, VA, USA) were cultivated in Dulbecco's modified eagle's medium (DMEM, Biowest, Nuaillé, France), 10 % fetal calf serum (FCS, c.c.pro, Oberdorla, Germany), 1 % sodium pyruvate (Sigma, Steinheim, Germany), 1 % non-essential amino acids (NEAA, Biowest) and 1 % penicillin/streptomycin (Biowest). For propagation of Hela cells (ACC 57, DSMZ, Braunschweig, Germany) DMEM media was supplemented with 10 % FCS, 1 % NEAA, 1 % penicillin/streptomycin and 1 % L-Glutamin (Biowest). Both cell lines were incubated at 37 °C and 5 % CO₂ in a humidified atmosphere.

### Dual luciferase reporter assay

Hela cells (10⁵) were seeded in 24 well plates, and 250 ng of the reporter plasmid psiCheck2 SARS-CoV-2 5'-UTR was co-transfected with 40 nM siRNA or 250 ng of the respective shRNA plasmid 24 h later using Lipofectamin 2000 (Thermo Fisher Scientific, Waltham, MA, USA) according to the manufacturer's instructions. Silencing efficacy of the siRNAs was determined after 48 h by measuring relative luciferase activity using the Dual-Luciferase Reporter Assay System (Promega) according to the manufacturer's protocol.

### Viral replication assay

All SARS-CoV-related infection experiments were performed under biosafety level-3 (BSL-3) conditions with enhanced respiratory personal protection equipment at Charité-Universitätsmedizin Berlin and Freie Universität Berlin. Vero E6 cells (3×10⁵ cells/mL) were transfected with 0.1 - 150 nM siCoV6 or 100 nM siCon 24 h after seeding using Lipofectamin RNAiMAX (Thermo Fisher Scientific) according to the manufacturer's protocol. Cells were washed with PBS 24 h after transfection and infected with SARS-CoV-2 (BetaCoV/Munich/BavPat2-ChVir984-ChVir1017/2020), SARS-CoV-2 alpha variant (BetaCoV/Germany/ChVir21652/2020), established by Daniela Niemeyer and Christian Drosten (Charité), or SARS-CoV-1 (recombinant SARS-CoV-1, GenBank: AY310120.1 (Pfefferle et al., 2009)), respectively at a MOI of 0.01. Viral RNA was extracted from 50 µL culture supernatant at the indicated time points using viral RNA isolation kits (Macherey-Nagel, Analytik Jena, Germany) according to the manufacturer's protocol. Genome equivalents per mL (GE/mL) were determined by quantitative RT-PCR as previously reported (Niemeyer et al., 2018; Corman et al., 2020).

### Cytopathogenicity

Cells were fixed in 4 % formaldehyde (Roth, Karlsruhe, Germany) 48 h after infection for 24 h and the CPE of viral infection was documented with a Zeiss Observer. Z1 microscope (Zeiss, Jena, Germany).

### Statistical analyses

Statistical significance was analyzed by one-way ANOVA or Student's *t*-test using GraphPad Prism 8 software (La Jolla, CA, USA). All data were collected in triplicate, in at least three independent experiments and are represented as mean ± SEM. Statistical significance is shown as *p ≤ 0.05, **p ≤ 0.01, ***p ≤ 0.001, ****p ≤ 0.0001.

### DISCUSSION

The novel human coronavirus disease, COVID-19, caused by SARS-CoV-2 has developed into a devastating pandemic. Ongoing research aims to identify therapeutic strategies for the prevention and treatment of COVID-19. However, despite the approval of vaccines against SARS-CoV-2 and increasing immunization rate of the population, satisfactory treatment options for patients with severe disease courses are still lacking (Jomah et al., 2020). Targeting gene expression by RNAi has become a standard method in biomedical research and the technology is being developed as a new therapeutic strategy with a growing number of siRNAs being approved by the authorities. It is also an auspicious approach to inhibit viral replication as frequently investigated in the past (Haasnoot and Berkhout, 2009; Levanova and Poranen, 2018). In particular, RNA viruses, like SARS-CoV-2, are promising candidates, as not only their mRNAs (as in case of DNA viruses), but also their genomic RNA can be directly targeted by RNAi. In accordance with this, the inventors and others could show in the past that the replication of RNA viruses, like the human immunodeficiency virus (Berkhout, 2009), influenza virus (Suzuki et al., 2009), coxsackievirus B3 (Werk et al., 2005; Stein et al., 2015) and hepatitis C virus (Chandra et al., 2012), was effectively inhibited by RNAi in various cell and animal disease models.

In the present study, the inventors designed siRNAs directed against the 5'-UTR of the SARS-CoV-2 genome by selecting a conserved region which is crucial for viral RNA replication and transcription, which previous experience has shown is a promising target (Yang and Leibowitz, 2015; Baldassarre et al., 2020; Pandey and Verma, 2021). As the efficiency of siRNAs depends on numerous factors such as their thermodynamic design and the structure of the target site (Kurreck, 2006) and their efficiency cannot be predicted, the inventors designed a set of eight siRNAs. Reporter assays confirmed that all eight designed siRNAs efficiently targeted the 5'-UTR of SARS-CoV-2 (Figure 1B) and led to a significant downregulation of the luciferase reporter activity (Figure 2B). The most efficient siRNA out of this panel of tested siRNAs, named siCoV6, binds to the highly conserved TRS and therefore targets the leader sequence. The leader sequence, consisting of around 75 nucleotides at the 5'-end of the SARS-CoV-2 genome, is also incorporated into the subgenomic RNAs by a process of discontinuous transcription. This unusual mechanism of virus protein biosynthesis makes the leader sequence an attractive target, as siRNAs will not only destroy the genomic RNA of SARS-CoV-2 but also the subgenomic RNAs (Figure 1A), which are essential for the translation of the structural proteins and hence the replication of the virus (Kim et al., 2020). In fact, siCoV6 effectively inhibited replication of SARS-CoV-2 and protected cells from a CPE (Figure 3). Moreover, as its target sequence is highly conserved, siCoV6 also showed promising inhibitory activity against the SARS-CoV-2 alpha variant and even against SARS-CoV-1 (Figure 4).

A challenge for the development of siRNA therapeutics with antiviral activity is that viruses mutate rather quickly, which frequently allows them to alter the target sequence of the siRNAs and severely reduce or completely abolish the efficiency of RNAi (Boden et al., 2003; Das et al., 2004; Wilson and Richardson, 2005). After the outbreak of SARS-CoV-1, several *in vitro* studies investigated siRNAs directed against the S protein of SARS-CoV-1 due to its fundamental role in cell infection (He et al., 2003; Qin et al., 2004; Zhang et al., 2004; Wu et al., 2005; Akerström et al., 2007). Moreover, the combination of two siRNAs targeting the S and N proteins also potently suppressed SARS-CoV-1-induced pathogenesis *in vivo* as shown in a Rhesus macaque SARS-CoV model (Li et al., 2005a). However, the S and N proteins are prone to mutations that do not reduce viral fitness, which is also observed in SARS-CoV-2 by an increasing number of SARS-CoV-2 variants (Jo et al., 2021). To prevent escape mutants it is therefore desirable to target a highly conserved region like the leader sequence, as demonstrated in a study by Li *et al.* in which an siRNA directed against the leader sequence of SARS-CoV-1 significantly inhibited virus replication in Vero E6 cells (Li et al., 2005b). They showed that the siRNA inhibited the expression of all the viral genes (S, E, M and N) and was more effective than an siRNA targeting the S gene alone. The target site of this siRNA directed against the leader sequence of SARS-CoV-1 is, however, mutated in the SARS-CoV-2 genome at two positions including one in the seed region, which can be expected to render the siRNA inefficient. In contrast, the siRNA siCoV6 designed in the present study targets a highly conserved sequence that is unaltered in the SARS-CoV-2 alpha variant and SARS-CoV-1 genomes.

A slight reduction in the efficiency of siCoV6 against the SARS-CoV-2 alpha variant (Figure 4A) can be explained by possible differences in the local target structure rather than mutations in the target site (Overhoff et al., 2005; Schubert et al., 2005b; Liu et al., 2013). When analyzing approximately 1.500 of the most recent complete sequences of the different SARS-CoV-2 variants (NCBI Virus SARS-CoV-2 Data Hub), the inventors did not find any mutations in the siCoV6 target site for the SARS-CoV-2 delta variant and the SARS-CoV-2 beta variant. For the SARS-CoV-2 alpha variant and the SARS-CoV-2 gamma variant only < 0.8 % of sequences showed mutations in the siCoV6 target site. The seed region of siCoV6 was, however, never affected. As the inventors have experimentally shown for the alpha variant as an example that siCoV6 maintains its activity, this bioinformatic analysis confirms the eligibility of the 5'-UTR and in particular the leader sequence as a target site for the implementation of RNAi as a broad-range antiviral approach (Baldassarre et al., 2020; Pandey and Verma, 2021).

Several other *in silico* studies predicted siRNAs targeting ORF1ab (Chen et al., 2020; Hasan et al., 2021; Niktab et al., 2021; Shawan et al., 2021), S gene (Chen et al., 2020; Panda et al., 2020; Chowdhury et al., 2021; Niktab et al., 2021), ORF3 (Chen et al., 2020; Niktab et al., 2021), E gene (Niktab et al., 2021), M gene (Chen et al., 2020; Niktab et al., 2021) and N gene (Chen et al., 2020; Chowdhury et al., 2021) of SARS-CoV-2. Two *in vitro* studies furthermore demonstrated a robust downregulation of S, M and N on the mRNA or protein levels by siRNAs in overexpression cell culture models (Gallicano et al., 2020; Wu and Luo, 2021). In a recent *in vivo* study by Idris *et al.*, siRNAs targeting the RdRp and helicase of SARS-CoV-2 were successfully delivered by lipid nanoparticle (LNP) formulations to the lung of a K18-hACE2 mouse model by intravenous injection. The siRNA-LNPs robustly repressed virus in the lungs and provided a survival advantage to the treated mice (Idris et al., 2021). Another recent *in vivo* study by Khaitov *et al.* demonstrated that topical application by inhalation of a stabilized siRNA modified with a peptide dendrimer targeting the RdRp of SARS-CoV-2 significantly reduced viral load and inflammation in the lung of a Syrian hamster model (Khaitov et al., 2021). Taken together, these are promising results for the development of RNAi-based therapy of COVID-19. However, as previously mentioned, targets like the S Protein or RdRp have the drawback that they are prone to mutations (Biswas and Mudi, 2020; Pachetti et al., 2020). The target site of the most promising siRNA designed by the inventors is the leader sequence that is highly conserved and enables the simultaneous targeting of the genomic RNA and all subgenomic RNAs. In analogy to combination therapy with low molecular weight drugs, potential viral escape can be suppressed with a combination of siRNAs (Schubert et al., 2005a; ter Brake et al., 2006; Merl and Wessely, 2007), for example, of siCoV6 with siCoV7, which also binds in a highly conserved region (Figure 1B). Though given the very high level of conservation of the leader sequence in SARS-CoV-2 and SARS-CoV-1, the viral escape potential is probably very low.

In the experiments commercial Lipofectamin RNAiMAX was used for siRNA transfection which is not recommended for *in vivo* use. The lung is the major site of a COVID-19-infection due to high expression of ACE2 and long-term lung damage can be a severe complication of COVID-19 (Zou et al., 2020). In the past few decades substantial progress has been made regarding the improvement of siRNA stability and efficiency of delivery to the respiratory system. (Durcan et al., 2008; DeVincenzo, 2012; Kaczmarek et al., 2017; Levanova and Poranen, 2018; Hu et al., 2020). For example, nasally applied siRNAs resulted in the highly effective inhibition of respiratory syncytial virus (RSV) and SARS-CoV-1 in animal models. (Bitko et al., 2005; Li et al., 2005a; Khaitov et al., 2014). Nebulizers or inhalers were developed to generate aerosols for drug delivery by inhalation directly to the lung (Youngren-Ortiz et al., 2016) as recently successfully implemented for SARS-CoV-2 inhibition (Khaitov et al., 2021). Administration of an siRNA targeting the RSV N gene (ALN-RSV01) by inhalation for the potential treatment or prevention of RSV infection was also investigated in a clinical study. The siRNA and application route were well tolerated and may have beneficial effects and prevent *bronchiolitis obliterans* syndrome in lung transplant patients infected with RSV (DeVincenzo et al., 2010; Zamora et al., 2011; Gottlieb et al., 2016). The inventors therefore envision delivery of siCoV6 to the lung by inhalation as a plausible administration route for the treatment of COVID-19.

A special feature of coronaviruses among RNA viruses is the existence of a proofreading mechanism, which hampers the development of nucleoside analogs as therapeutics as they are removed during replication and transcription (Robson et al., 2020). At the same time, the proofreading activity is favorable for RNA targeting approaches as it increases genomic stability thereby reducing the risk of the development of escape mutants. The inventors demonstrated the high antiviral activity of siCoV6 against SARS-CoV-2. Its target site is widely conserved among the currently available SARS-CoV-2 variants, and the inventors experimentally demonstrated that the siRNA maintains its activity against SARS-CoV-2 alpha and even against SARS-CoV-1. This broad antiviral activity makes siCoV6 a promising candidate for the treatment of SARS-coronavirus infections.

### List of references cited in the preceding sections

1. Akerström, S., Mirazimi, A., and Tan, Y.-J. (2007). Inhibition of SARS-CoV replication cycle by small interference RNAs silencing specific SARS proteins, 7a/7b, 3a/3b and S. Antiviral Research 73, 219-227.
2. Baldassarre, A., Paolini, A., Bruno, S.P., Felli, C., Tozzi, A.E., and Masotti, A. (2020). Potential use of noncoding RNAs and innovative therapeutic strategies to target the 5'UTR of SARS-CoV-2. Epigenomics.
3. Bantounas, I., Phylactou, L.A., and Uney, J.B. (2004). RNA interference and the use of small interfering RNA to study gene function in mammalian systems. Journal of Molecular Endocrinology 33, 545-557.
4. Berkhout, B. (2009). Toward a durable anti-HIV gene therapy based on RNA interference. Annals of the New York Academy of Sciences 1175, 3-14.
5. Biswas, S.K., and Mudi, S.R. (2020). Spike protein D614G and RdRp P323L: the SARS-CoV-2 mutations associated with severity of COVID-19. Genomics & informatics 18, e44.
6. Bitko, V., Musiyenko, A., Shulyayeva, O., and Barik, S. (2005). Inhibition of respiratory viruses by nasally administered siRNA. Nature medicine 11, 50-55.
7. Boden, D., Pusch, O., Lee, F., Tucker, L., and Ramratnam, B. (2003). Human immunodeficiency virus type 1 escape from RNA interference. Journal of Virology 77, 11531-11535.
8. Chan, J.F.-W., Kok, K.-H., Zhu, Z., Chu, H., To, K.K.-W., Yuan, S., and Yuen, K.-Y. (2020). Genomic characterization of the 2019 novel human-pathogenic coronavirus isolated from a patient with atypical pneumonia after visiting Wuhan. Emerging Microbes & Infections 9, 221-236.
9. Chandra, P.K., Kundu, A.K., Hazari, S., Chandra, S., Bao, L., Ooms, T., Morris, G.F., Wu, T., Mandal, T.K., and Dash, S. (2012). Inhibition of hepatitis C virus replication by intracellular delivery of multiple siRNAs by nanosomes. Molecular Therapy 20, 1724-1736.
10. Chang, Z., Babiuk, L.A., and Hu, J. (2007). Therapeutic and prophylactic potential of small interfering RNAs against severe acute respiratory syndrome: progress to date. BioDrugs : clinical immunotherapeutics, biopharmaceuticals and gene therapy 21, 9-15.
11. Chen, W., Feng, P., Liu, K., Wu, M., and Lin, H. (2020). Computational Identification of Small Interfering RNA Targets in SARS-CoV-2. Virologica Sinica, 1-3.
12. Chen, Y., Cheng, G., and Mahato, R.I. (2008). RNAi for treating hepatitis B viral infection. Pharmaceutical research 25, 72-86.
13. Chowdhury, U.F., Sharif Shohan, M.U., Hoque, K.I., Beg, M.A., Sharif Siam, M.K., and Moni, M.A. (2021). A computational approach to design potential siRNA molecules as a prospective tool for silencing nucleocapsid phosphoprotein and surface glycoprotein gene of SARS-CoV-2. Genomics 113, 331-343.
14. Corman, V.M., Landt, O., Kaiser, M., Molenkamp, R., Meijer, A., Chu, D.K., Bleicker, T., Brünink, S., Schneider, J., and Schmidt, M.L., et al. (2020). Detection of 2019 novel coronavirus (2019-nCoV) by real-time RT-PCR. Euro surveillance : bulletin Europeen sur les maladies transmissibles = European communicable disease bulletin 25*.*
15. Das, A.T., Brummelkamp, T.R., Westerhout, E.M., Vink, M., Madiredjo, M., Bernards, R., and Berkhout, B. (2004). Human immunodeficiency virus type 1 escapes from RNA interference-mediated inhibition. Journal of Virology 78, 2601-2605.
16. DeVincenzo, J., Lambkin-Williams, R., Wilkinson, T., Cehelsky, J., Nochur, S., Walsh, E., Meyers, R., Gollob, J., and Vaishnaw, A. (2010). A randomized, double-blind, placebo-controlled study of an RNAi-based therapy directed against respiratory syncytial virus. PNAS 107, 8800-8805.
17. DeVincenzo, J.P. (2012). The promise, pitfalls and progress of RNA-interference-based antiviral therapy for respiratory viruses. Antiviral therapy 17, 213-225.
18. Durcan, N., Murphy, C., and Cryan, S.-A. (2008). Inhalable siRNA: potential as a therapeutic agent in the lungs. Molecular pharmaceutics 5, 559-566.
19. Eckstein, A., Grössl, T., Geisler, A., Wang, X., Pinkert, S., Pozzuto, T., Schwer, C., Kurreck, J., Weger, S., and Vetter, R., et al. (2010). Inhibition of adenovirus infections by siRNA-mediated silencing of early and late adenoviral gene functions. Antiviral Research 88, 86-94.
20. Gallicano, G.I., Casey, J.L., Fu, J., and Mahapatra, S. (2020). Molecular targeting of vulnerable RNA sequences in SARS CoV-2: identifying clinical feasibility. Gene Ther, 1-8.
21. Gottlieb, J., Zamora, M.R., Hodges, T., Musk, A.W., Sommerwerk, U., Dilling, D., Arcasoy, S., DeVincenzo, J., Karsten, V., and Shah, S., et al. (2016). ALN-RSV01 for prevention of bronchiolitis obliterans syndrome after respiratory syncytial virus infection in lung transplant recipients. The Journal of heart and lung transplantation : the official publication of the International Society for Heart Transplantation 35, 213-221.
22. Haasnoot, J., and Berkhout, B. (2009). Nucleic acids-based therapeutics in the battle against pathogenic viruses. Handbook of experimental pharmacology 189, 243-263.
23. Hasan, M., Ashik, A.I., Chowdhury, M.B., Tasnim, A.T., Nishat, Z.S., Hossain, T., and Ahmed, S. (2021). Computational prediction of potential siRNA and human miRNA sequences to silence orf1ab associated genes for future therapeutics against SARS-CoV-2. Informatics in medicine unlocked 24, 100569.
24. He, M.-L., Zheng, B., Peng, Y., Peiris, J.S.M., Poon, L.L.M., Yuen, K.Y., Lin, M.C.M., Kung, H.-f., and Guan, Y. (2003). Inhibition of SARS-associated coronavirus infection and replication by RNA interference. JAMA 290, 2665-2666.
25. Hoffmann, M., Kleine-Weber, H., Schroeder, S., Krüger, N., Herrler, T., Erichsen, S., Schiergens, T.S., Herrler, G., Wu, N.-H., and Nitsche, A., et al. (2020). SARS-CoV-2 Cell Entry Depends on ACE2 and TMPRSS2 and Is Blocked by a Clinically Proven Protease Inhibitor. Cell 181, 271-280.e8.
26. Hu, B., Zhong, L., Weng, Y., Peng, L., Huang, Y., Zhao, Y., and Liang, X.-J. (2020). Therapeutic siRNA: state of the art. Sig Transduct Target Ther 5, 101.
27. Idris, A., Davis, A., Supramaniam, A., Acharya, D., Kelly, G., Tayyar, Y., West, N., Zhang, P., McMillan, C.L.D., and Soemardy, C., et al. (2021). A SARS-CoV-2 targeted siRNA-nanoparticle therapy for COVID-19. bioRxiv : the preprint server for biology.
28. Jo, W.K., Drosten, C., and Drexler, J.F. (2021). The evolutionary dynamics of endemic human coronaviruses. Virus evolution 7, veab020.
29. Jomah, S., Asdaq, S.M.B., and Al-Yamani, M.J. (2020). Clinical efficacy of antivirals against novel coronavirus (COVID-19): A review. Journal of infection and public health 13, 1187-1195.
30. Kaczmarek, J.C., Kowalski, P.S., and Anderson, D.G. (2017). Advances in the delivery of RNA therapeutics: from concept to clinical reality. Genome medicine 9, 60.
31. Khaitov, M., Nikonova, A., Shilovskiy, I., Kozhikhova, K., Kofiadi, I., Vishnyakova, L., Nikolsky, A., Gattinger, P., Kovchina, V., and Barvinskaya, E., et al. (2021). Silencing of SARS-CoV-2 with modified siRNA-peptide dendrimer formulation. Allergy.
32. Khaitov, M.R., Shilovskiy, I.P., Nikonova, A.A., Shershakova, N.N., Kamyshnikov, O.Y., Babakhin, A.A., Zverev, V.V., Johnston, S.L., and Khaitov, R.M. (2014). Small interfering RNAs targeted to interleukin-4 and respiratory syncytial virus reduce airway inflammation in a mouse model of virus-induced asthma exacerbation. Human gene therapy 25, 642-650.
33. Kim, D., Lee, J.-Y., Yang, J.-S., Kim, J.W., Kim, V.N., and Chang, H. (2020). The Architecture of SARS-CoV-2 Transcriptome. Cell 181, 914-921.e10.
34. Kurreck, J. (2006). siRNA efficiency: structure or sequence-that is the question. Journal of biomedicine & biotechnology 2006, 83757.
35. Kurreck, J. (2009). RNA interference: from basic research to therapeutic applications. Angewandte Chemie International Edition 48, 1378-1398.
36. Levanova, A., and Poranen, M.M. (2018). RNA Interference as a Prospective Tool for the Control of Human Viral Infections. Front. Microbiol. 9, 2151.
37. Li, B.-j., Tang, Q., Du Cheng, Qin, C., Xie, F.Y., Wei, Q., Xu, J., Liu, Y., Zheng, B.-J., and Woodle, M.C., et al. (2005a). Using siRNA in prophylactic and therapeutic regimens against SARS coronavirus in Rhesus macaque. Nature medicine 11, 944-951.
38. Li, T., Zhang, Y., Fu, L., Yu, C., Li, X., Li, Y., Zhang, X., Rong, Z., Wang, Y., and Ning, H., et al. (2005b). siRNA targeting the leader sequence of SARS-CoV inhibits virus replication. Gene Ther 12, 751-761.
39. Liu, Y., Chang, Y., Zhang, C., Wei, Q., Chen, J., Chen, H., and Xu, D. (2013). Influence of mRNA features on siRNA interference efficacy. Journal of bioinformatics and computational biology 11, 1341004.
40. Merl, S., and Wessely, R. (2007). Anti-coxsackieviral efficacy of RNA interference is highly dependent on genomic target selection and emergence of escape mutants. Oligonucleotides 17, 44-53.
41. Niemeyer, D., Mösbauer, K., Klein, E.M., Sieberg, A., Mettelman, R.C., Mielech, A.M., Dijkman, R., Baker, S.C., Drosten, C., and Müller, M.A. (2018). The papain-like protease determines a virulence trait that varies among members of the SARS-coronavirus species. PLoS Pathogens 14, e1007296.
42. Niktab, I., Haghparast, M., Beigi, M.-H., Megraw, T.L., Kiani, A., and Ghaedi, K. (2021). Design of advanced siRNA therapeutics for the treatment of COVID-19. Meta gene 29, 100910.
43. Overhoff, M., Alken, M., Far, R.K.-K., Lemaitre, M., Lebleu, B., Sczakiel, G., and Robbins, I. (2005). Local RNA target structure influences siRNA efficacy: a systematic global analysis. Journal of molecular biology 348, 871-881.
44. Pachetti, M., Marini, B., Benedetti, F., Giudici, F., Mauro, E., Storici, P., Masciovecchio, C., Angeletti, S., Ciccozzi, M., and Gallo, R.C., et al. (2020). Emerging SARS-CoV-2 mutation hot spots include a novel RNA-dependent-RNA polymerase variant. Journal of translational medicine 18, 179.
45. Pal, M., Berhanu, G., Desalegn, C., and Kandi, V. (2020). Severe Acute Respiratory Syndrome Coronavirus-2 (SARS-CoV-2): An Update. Cureus 12, e7423.
46. Panda, K., Alagarasu, K., Cherian, S.S., and Parashar, D. (2020). Prediction of potential small interfering RNA molecules for silencing of the spike gene of SARS-CoV-2. The Indian journal of medical research 153, 182.
47. Pandey, A.K., and Verma, S. (2021). An in silico analysis of effective siRNAs against COVID-19 by targeting the leader sequence of SARS-CoV-2. Advances in Cell and Gene Therapy, e107.
48. Pfefferle, S., Krähling, V., Ditt, V., Grywna, K., Mühlberger, E., and Drosten, C. (2009). Reverse genetic characterization of the natural genomic deletion in SARS-Coronavirus strain Frankfurt-1 open reading frame 7b reveals an attenuating function of the 7b protein in-vitro and in-vivo. Virol J 6, 131.
49. Pruijssers, A.J., George, A.S., Schäfer, A., Leist, S.R., Gralinksi, L.E., Dinnon, K.H., Yount, B.L., Agostini, M.L., Stevens, L.J., and Chappell, J.D., et al. (2020). Remdesivir Inhibits SARS-CoV-2 in Human Lung Cells and Chimeric SARS-CoV Expressing the SARS-CoV-2 RNA Polymerase in Mice. Cell Reports 32, 107940.
50. Qin, Z.-I., Zhao, P., Zhang, X.-I., Yu, J.-g., Cao, M.-m., Zhao, L.-j., Luan, J., and Qi, Z.-t. (2004). Silencing of SARS-CoV spike gene by small interfering RNA in HEK 293T cells. Biochemical and biophysical research communications 324, 1186-1193.
51. Robson, F., Khan, K.S., Le, T.K., Paris, C., Demirbag, S., Barfuss, P., Rocchi, P., and Ng, W.-L. (2020). Coronavirus RNA Proofreading: Molecular Basis and Therapeutic Targeting. Molecular cell 79, 710-727.
52. Romano, M., Ruggiero, A., Squeglia, F., Maga, G., and Berisio, R. (2020). A Structural View of SARS-CoV-2 RNA Replication Machinery: RNA Synthesis, Proofreading and Final Capping. Cells 9, 1267.
53. Sawicki, S.G., and Sawicki, D.L. (1998). A new model for coronavirus transcription. Advances in experimental medicine and biology 440, 215-219.
54. Schaar, K., Geisler, A., Kraus, M., Pinkert, S., Pryshliak, M., Spencer, J.F., Tollefson, A.E., Ying, B., Kurreck, J., and Wold, W.S., et al. (2017). Anti-adenoviral Artificial MicroRNAs Expressed from AAV9 Vectors Inhibit Human Adenovirus Infection in Immunosuppressed Syrian Hamsters. Molecular therapy. Nucleic acids 8, 300-316.
55. Schoeman, D., and Fielding, B.C. (2019). Coronavirus envelope protein: current knowledge. Virol J 16, 69*.*
56. Schubert, S., Grunert, H.-P., Zeichhardt, H., Werk, D., Erdmann, V.A., and Kurreck, J. (2005a). Maintaining inhibition: siRNA double expression vectors against coxsackieviral RNAs. Journal of molecular biology 346, 457-465.
57. Schubert, S., Grünweller, A., Erdmann, V.A., and Kurreck, J. (2005b). Local RNA target structure influences siRNA efficacy: systematic analysis of intentionally designed binding regions. Journal of molecular biology 348, 883-893.
58. Shawan, M.M.A.K., Sharma, A.R., Bhattacharya, M., Mallik, B., Akhter, F., Shakil, M.S., Hossain, M.M., Banik, S., Lee, S.-S., and Hasan, M.A., et al. (2021). Designing an effective therapeutic siRNA to silence RdRp gene of SARS-CoV-2. Infection, Genetics and Evolution 93, 104951.
59. Stein, E.A., Pinkert, S., Becher, P.M., Geisler, A., Zeichhardt, H., Klopfleisch, R., Poller, W., Tschöpe, C., Lassner, D., and Fechner, H., et al. (2015). Combination of RNA interference and virus receptor trap exerts additive antiviral activity in coxsackievirus B3-induced myocarditis in mice. J Infect Dis 211, 613-622.
60. Suzuki, H., Saitoh, H., Suzuki, T., and Takaku, H. (2009). Baculovirus-mediated bispecific short-hairpin small-interfering RNAs have remarkable ability to cope with both influenza viruses A and B. Oligonucleotides 19, 307-316.
61. ter Brake, O., Konstantinova, P., Ceylan, M., and Berkhout, B. (2006). Silencing of HIV-1 with RNA interference: a multiple shRNA approach. Molecular Therapy 14, 883-892.
62. Walls, A.C., Park, Y.-J., Tortorici, M.A., Wall, A., McGuire, A.T., and Veesler, D. (2020). Structure, Function, and Antigenicity of the SARS-CoV-2 Spike Glycoprotein. Cell 181, 281-292.e6.
63. Wan, J., Xing, S., Ding, L., Wang, Y., Gu, C., Wu, Y., Rong, B., Li, C., Wang, S., and Chen, K., et al. (2020). Human-IgG-Neutralizing Monoclonal Antibodies Block the SARS-CoV-2 Infection. Cell Reports 32, 107918.
64. Werk, D., Schubert, S., Lindig, V., Grunert, H.-P., Zeichhardt, H., Erdmann, V.A., and Kurreck, J. (2005). Developing an effective RNA interference strategy against a plus-strand RNA virus: silencing of coxsackievirus B3 and its cognate coxsackievirus-adenovirus receptor. Biological Chemistry 386, 857-863.
65. Wilson, J.A., and Richardson, C.D. (2005). Hepatitis C virus replicons escape RNA interference induced by a short interfering RNA directed against the NS5b coding region. Journal of Virology 79, 7050-7058.
66. Wu, C.-J., Huang, H.-W., Liu, C.-Y., Hong, C.-F., and Chan, Y.-L. (2005). Inhibition of SARS-CoV replication by siRNA. Antiviral Research 65, 45-48.
67. Wu, R., and Luo, K.Q. (2021). Developing effective siRNAs to reduce the expression of key viral genes of COVID-19. International journal of biological sciences 17, 1521-1529.
68. Yang, D., and Leibowitz, J.L. (2015). The structure and functions of coronavirus genomic 3' and 5' ends. Virus Research 206, 120-133.
69. Youngren-Ortiz, S.R., Gandhi, N.S., España-Serrano, L., and Chougule, M.B. (2016). Aerosol Delivery of siRNA to the Lungs. Part 1: Rationale for Gene Delivery Systems. Kona : powder science and technology in Japan 33, 63-85.
70. Zamora, M.R., Budev, M., Rolfe, M., Gottlieb, J., Humar, A., DeVincenzo, J., Vaishnaw, A., Cehelsky, J., Albert, G., and Nochur, S., et al. (2011). RNA interference therapy in lung transplant patients infected with respiratory syncytial virus. American journal of respiratory and critical care medicine 183, 531-538.
71. Zhang, Y., Li, T., Fu, L., Yu, C., Li, Y., Xu, X., Wang, Y., Ning, H., Zhang, S., and Chen, W., et al. (2004). Silencing SARS-CoV Spike protein expression in cultured cells by RNA interference. Febs Letters 560, 141-146.
72. Zou, X., Chen, K., Zou, J., Han, P., Hao, J., and Han, Z. (2020). Single-cell RNA-seq data analysis on the receptor ACE2 expression reveals the potential risk of different human organs vulnerable to 2019-nCoV infection. Frontiers of medicine 14, 185-192.

## Claims

1. RNA for inhibiting severe acute respiratory syndrome coronavirus 2 (SARS-CoV-2) replication, **characterized in that** the RNA is an at least partially double-stranded RNA having a sequence being at least 95 % identical to a sequence chosen from the group consisting of SEQ ID NO. 6 and SEQ ID NO. 11.

2. RNA according to claim 1 for use as a medicament.

3. RNA according to claim 1 for use according to claim 2, **characterized in that** the medicament is an inhalation medicament.

4. RNA according to claim 1 for use in treating a disease caused by a coronavirus, wherein the coronavirus has a genome or subgenome comprising an RNA sequence being at least 95 % identical or complementary to a sequence chosen from the group consisting of SEQ ID NO. 6 and SEQ ID NO. 11.

5. RNA according to claim 1 for use according to claim 4, **characterized in that** the coronavirus is a severe acute respiratory syndrome coronavirus (SARS-CoV).

6. RNA according to claim 1 for use according to claim 4 or 5, **characterized in that** the disease is coronavirus disease 2019 (COVID-19).

7. In-vitro method for inhibiting SARS-CoV-2 replication in an isolated cell, **characterized in that** the method comprises transfecting the isolated cell with an RNA according to claim 1.

8. Pharmaceutical preparation, comprising at least one RNA according to claim 1.

9. Pharmaceutical preparation according to claim 8, **characterized in that** the pharmaceutical preparation additionally comprises an RNA having a sequence being at least 95 % identical to a sequence chosen from the group consisting of SEQ ID NO. 1, SEQ ID NO. 2, SEQ ID NO. 3, SEQ ID NO. 4, SEQ ID NO. 5, SEQ ID NO. 7, SEQ ID NO. 8, SEQ ID NO. 10, and SEQ ID NO. 12.

10. Pharmaceutical preparation according to claim 8 or 9, **characterized in that** the pharmaceutical preparation comprises an RNA having a sequence being at least 95 % identical to SEQ ID NO. 11 and an RNA having a sequence being at least 95 % identical to a sequence chosen from the group consisting of SEQ ID NO. 10 and SEQ ID NO. 12.

11. Pharmaceutical preparation according to any of claims 8 to 10, **characterized in that** the pharmaceutical preparation further comprises an adeno-associated virus.

12. A method for treating or preventing a disease caused by a coronavirus in a person in need thereof, wherein the coronavirus has a genome or subgenome comprising an RNA sequence being at least 95 % identical or complementary to a sequence chosen from the group consisting of SEQ ID NO. 6 and SEQ ID NO. 11, the method comprising administering to the person a pharmaceutically active amount of an at least partially double-stranded RNA having a sequence being at least 95 % identical to a sequence chosen from the group consisting of SEQ ID NO. 6 and SEQ ID NO. 11.
